# EUROPEAN PATENT APPLICATION

(11) **EP 4 464 723 A1**
(43) Date of publication of application: **20.11.2024**
(21) Application number: 23740491.8
(22) Date of filing: 13.01.2023
(51) Int. Cl.: C07K 16/28, C12N 15/62, C12N 15/63, A61P 35/00, G01N 33/68

(54) **FUSION PROTEIN COMPRISING MODIFIED ANTI-VEGFR2 (KDR) ANTIBODY AND USE THEREOF**

(30) Priority: 14.01.2022 KR 20220005625
(71) Applicant: Pharmabcine Inc., Daejeon 34047 (KR)
(72) Inventor: NAM, Ju Ryoung, Daejeon 34047 (KR); LEE, Youngae, Daejeon 34047 (KR); LEE, Weon Sup, Daejeon 34047 (KR); YOO, Jin-San, Daejeon 34047 (KR)
(74) Representative: Mewburn Ellis LLP
(86) International application number: PCT/KR2023/000631
(87) International publication number: WO 2023/136648

(57) **Abstract**

The present invention relates to a fusion protein comprising a modified anti-VEGFR2 (KDR) antibody having improved properties or an antigen-binding fragment thereof, and a use thereof. Specifically, the present invention pertains to a fusion protein, and a composition for diagnosis or treatment of an angiogenesis-associated disease, a composition for prevention or treatment of a tumor or cancer, and a composition for administration in combination with a therapeutic agent other than the fusion protein, each composition comprising the fusion protein, wherein the fusion protein has an anti-VEGFR2(KDR) antibody conjugated with a delta-like ligand 4 (DLL4)-binding protein domain at the terminus thereof, the anti-VEGFR2(KDR) antibody being derived from a human antibody binding specifically to VEGFR2/KDR by substituting VH1 in the heavy chain framework region (FR) with VH3 and VL1 in the light chain framework region (FR) with VK1 and inducing a mutation therein, thereby increasing the affinity of the antibody for the antigen.

## Description

### [Technical Field]

The present invention relates to a fusion protein including a modified anti-VEGFR2 (KDR) antibody exhibiting improved properties or an antigen-binding fragment thereof, and the use thereof. More particularly, the present invention relates to a fusion protein including an anti-VEGFR2 (KDR) antibody and a DLL4 (delta-like ligand 4) binding protein domain bound to the terminus thereof, the anti-VEGFR2 (KDR) antibody having increased affinity for an antigen by substituting VH1 in the heavy chain FR (framework region) of a human antibody that specifically binds to VEGFR2/KDR with VH3 and VL1 in the light chain FR (framework region) with VK1 and inducing mutations therein, and to a composition for diagnosing or treating an angiogenesis-related disease, a composition for preventing or treating a tumor or cancer, and a composition for co-administration with a therapeutic agent in addition to the fusion protein, each composition including the fusion protein.

### [Background Art]

VEGFR2/KDR is the primary angiogenic receptor, binds to VEGF isoforms A, C, D, and E, and is important not only for endothelial cell differentiation but also for the effects of enhancing mitogenesis, angiogenesis, and permeability of VEGF. Anti-VEGFR2 antibodies may prevent all known VEGF isoforms from binding to VEGFR2/KDR and initiating signaling. Also, since tumors secrete greater numbers of VEGF but the number of receptors remains relatively constant, targeting the receptor increases a probability of completely inhibiting signaling even in the presence of very high levels of VEGF isoforms.

Angiogenesis refers to the creation of new blood vessels from existing blood vessels by growth, division, migration, etc. of endothelial cells. In adults, angiogenesis does not occur except in special cases such as wound healing, female reproductive cycles, and the like. However, excessive angiogenesis has been reported in diseases, such as tumor growth and metastasis, age-related macular denaturation, rheumatoid arthritis, diabetic retinopathy, psoriasis, and chronic inflammation (Cameliet and Jain, Nature, 407:249, 2000), and for this reason, many efforts are being made to treat diseases, especially tumors, using angiogenesis inhibitors.

Since the hypothesis was raised by Dr. J. Folkman in 1971 that tumor growth and metastasis are dependent on angiogenesis and therefore that therapy focusing on anti-angiogenesis has the potential to become a new treatment for solid cancers, research related to the inhibition of excessive angiogenesis mechanisms has attracted the attention of many researchers (Ferrara and Kerbel, Nature, 435:967, 2005). The progression of angiogenesis is determined by comprehensive balance of angiogenesis-inducing factors and angiogenesis-inhibiting factors, and proceeds by a complex and sequential process of multiple stages. Specifically, various angiogenesis-inducing factors, including vascular endothelial growth factors (VEGFs) secreted from tumors or wound tissue, bind to the relevant receptors of existing vascular endothelial cells in the surrounding area, thereby activating vascular endothelial cells, so that the permeability of vascular endothelial cells increases, and by secreting proteolytic enzymes such as matrix metalloproteinase (MMP), the basement membrane and extracellular matrix around the vascular endothelial cells are decomposed, so that the vascular endothelial cells move away from existing capillaries and migrate and proliferate toward tissues that secrete angiogenesis-inducing factors. The migrating and proliferating vascular endothelial cells form a tube structure within the blood vessels, and finally, the influx of pericytes, which are structural supports for the vascular endothelial cells, leads to the formation of stable and mature blood vessels.

In the blood vessel formation process described above, one of the main factors for normal vascular tube formation is DLL4. DLL4 is specifically induced by VEGF and plays a role in appropriately regulating angiogenic sprouting by acting on Notch receptors. Blocking DLL4 results in loss of lateral inhibition in cells distal to the angiogenesis site, ultimately causing excessive sprouting. Thereby, the angiogenic response, which is excessive but low in productivity, increases and perfusion that supplies oxygen worsens, leading to a hypoxic state around cancer. In experimental cancer models, activation of DLL4 signaling improved angiogenesis, whereas inhibition thereof suppressed cancer growth (Lobov IB et al., Proc Natl Acad Sci U S A. 2007 Feb 27;104(9):3219-24).

DLL4 is one of the ligands for Notch receptors, and there are currently known four types of Notch receptors (Notch1-4) and five types of Notch ligands (Jagged-1, Jagged-2, DLL1, DLL3, and DLL4) in mammals. It is known that the Notch signaling system begins when the Notch ligand of one cell binds to the Notch receptor of another cell, and is activated only by direct interaction between different cells (Bray SJ, Nat Rev Mol Cell Biol., 7(9):678, 2006).

When a Notch ligand binds to a Notch receptor, ADAM metalloprotease is first activated to break the outer proximal portion of the cell membrane of the Notch receptor, and then the gamma-secretase complex is activated to break the inner proximal portion of the cell membrane of the Notch receptor, so that NICD (Notch intracellular domain) is released, and NICD moves to the nucleus and binds to RBPJ/CSL transcription factors to induce expression of Notch target genes such as basic helix-loop-helix proteins, for example Hes and Hey. In this way, the Notch signaling system determines the fate of proliferation/differentiation/apoptosis, etc. depending on the cell situation, and also plays an important role in maintaining normal and cancer stem cells.

Basically, all Notch receptors may bind to all Notch ligands, but the combination of these various bindings is selectively controlled in a microenvironment in which the cells are located. For example, DLL4 is strongly expressed in endothelial cells during angiogenesis in the course of fetal development and binds to Notch1 and Notch4 expressed in surrounding endothelial cells, but thereamong, DLL4-Notch1 binding is the most important (Yan M, Vasc Cell, 2011), and angiogenesis proceeds through this binding. This fact has been well revealed through gene deficiency experiments and the like (Duarte et al., Genes Dev, 2004; Gale et al., PNAS, 2004; Krebs et al., Genes Dev, 2004).

Therefore, inhibiting DLL4-Notch1 binding may suppress angiogenesis, thereby making it possible to treat various diseases such as tumors, etc. In cancer treatment, suppressing VEGF using Avastin (bevacizumab), etc. inhibits angiogenesis, decreasing tumor perfusion and thus reducing tumor size, while targeting DLL4 to inhibit binding to Notch1 expressed in surrounding cells results in hypersprouting of nonfunctional blood vessels, decreasing tumor perfusion and ultimately reducing tumor size (Thurston et al., Nat Rev Cancer, 7(5):327, 2007).

Therefore, the present research team has attempted to effectively inhibit angiogenesis by producing a dual-target antibody with Notch1, particularly the DLL4 binding site of Notch1, fused to an existing antibody.

Factors involved in angiogenesis include vascular endothelial growth factor (VEGF), epithelial growth factor (EGF), platelet-derived growth factor (PDGF), transforming growth factor (TGFb), fibroblast growth factor (FGF), and the like. Thereamong, endothelial growth factor is an endothelial cell-specific factor that is directly involved in growth, differentiation, and migration of endothelial cells, and includes four different isoforms (VEGF165, VEGF121, VEGF189, and VEGF206), among which VEGF165 is the most abundant isoform in all human tissues except the placenta (Tisher et al., J Biol Chem, 266: 11947, 1991).

Vascular endothelial growth factor (VEGF) regulates *in situ* the formation of new blood vessels through differentiation of endothelial precursors (hemangioblasts), is expressed in embryonic tissues (Breier et al., Development (Camb), 114:521, 1992), macrophages, and proliferating epithelial keratinocytes during wound healing (Brown et al., J. Exp. Med., 176:1375, 1992), and may be a cause of tissue edema associated with inflammation (Ferrara et al., Endocr. Rev., 13:18, 1992). *In situ* hybridization studies have demonstrated that VEGF is highly expressed in a number of human tumor lines, including glioblastoma multiforme, hemangioblastoma, central nervous system neoplasms, and AIDS-related Kaposi sarcoma (Plate et al., Nature 359:845,1992; Plate et al., Cancer Res. 53: 5822, 1993; Berkman et al., J. Clin. Invest. 91: 153, 1993; Nakamura et al., AIDS Weekly. 13(1), 1992). A high level of VEGF has also been observed in hypoxia-induced angiogenesis (Shweiki et al., Nature 359:843, 1992).

The biological functions of VEGF are mediated through the VEGF receptor, which has high affinity for VEGF, and the receptor is selectively expressed in endothelial cells during embryogenesis (Millauer et al., Cell, 72: 835, 1993) and tumorigenesis. In general, VEGF receptors (VEGFRs) are class III receptor-type tyrosine kinases characterized by having multiple, typically five or seven, immunoglobulin-like loops in their amino-terminal extracellular receptor ligand-binding domain (Kaipainen et al., J. Exp. Med., 178: 2027, 1993). The other two regions include a carboxy-terminal intracellular catalytic domain and a transmembrane region interrupted by the insertion of a hydrophilic interkinase sequence of variable length, termed the kinase insert domain (Terman et al., Oncogene, 6: 1677, 1991). Although other receptors such as neuropilin-1 and neuropilin-2 may also bind to VEGF, VEGFR includes fms-like tyrosine kinase receptor (flt-1), or VEGFR-1 (Shibuya et al., Oncogene, 5: 519, 1990; WO 92/14248; Terman et al., Oncogene, 6:1677, 1991) and kinase insert domain-containing receptor/fetal liver kinase (KDR/flk-1), or VEGFR-2 (Matthews et al., Proc. Natl. Acad. Sci. USA, 88:9026,1991). Another tyrosine kinase receptor, VEGFR-3 (flt-4), binds to the VEGF homologs VEGF-C and VEGF-D and plays an important role in the development of lymphatic vessels.

A high level of flk-1 is expressed by endothelial cells infiltrating gliomas (Plate et al., Nature 359:845, 1992). The flk-1 level is specifically upregulated by VEGF produced by human glioblastoma (Plate et al., Cancer Res. 53: 5822, 1993). The high level of expression of flk-1 in glioblastoma-associated endothelial cells (GAECs) indicates that receptor activity is probably induced during tumorigenesis because flk-1 transcripts are barely detectable in normal brain endothelial cells. This upregulation occurs only in vascular endothelial cells in close proximity to the tumor. Blocking VEGF activity with a neutralizing anti-VEGF monoclonal antibody (mAb) inhibits the growth of human tumor grafts in nude mice (Kim et al., Nature 362:841, 1993), indicating that VEGF plays a direct role in tumor-related angiogenesis.

Although VEGF ligands are upregulated in tumor cells and the receptors thereof are upregulated in tumor-infiltrating vascular endothelial cells, expression of VEGF ligands and receptors thereof is low in normal cells not associated with angiogenesis. Therefore, in these normal cells, angiogenesis is suppressed by blocking the interaction between VEGF and the receptor thereof, and thus no tumor growth occurs.

A high level of VEGFR-2 is expressed by endothelial cells infiltrating gliomas and is specifically upregulated by VEGF produced by human glioblastoma (Plate et al., Nature, 359:845, 1992; Plate et al., Cancer Res., 53:5822, 1993). Since VEGFR-2 transcripts are barely detectable in normal brain endothelial cells, the high level of VEGFR-2 expression in glioblastoma-associated endothelial cells (GAEC) indicates that receptor activity is induced during tumorigenesis.

VEGF is highly expressed in various types of tumors (Plate et al., Nature, 359:8435, 1992; Dvorak et al., J. Exp. Med., 174:1275, 1991), and newly formed capillaries gather around VEGF-producing tumor follicles (Plate et al., Nature, 359:8435, 1992). VEGF expression is strongly upregulated under hypoxic conditions, such as those associated with rapidly growing tumors (Shweiki et al., Nature, 359:843, 1992). VEGF neutralization by antibodies such as Avastin (Ferrara et al., Nat Rev Drug Discov., 3(5):391, 2004; Avery et al., Ophthalmology, 113(3):363, 2006) is a clinically approved therapy to inhibit cancer or other angiogenic diseases such as AMD. However, resistance to VEGF blockade was found even when combined with chemotherapy. This resistance may be associated with remodeled vasculature and increased expression of other angiogenic factors. Accordingly, there remains a need in the art for improved compositions and methods to inhibit cancer and other angiogenesis-related diseases.

Although both tyrosine kinase inhibitors (TKIs) and anti-KDRP antibodies may inhibit KDR-mediated angiogenesis, antibody approach has advantages over TKIs. In contrast to TKIs, anti-KDR antibodies are more specific KDR targeting agents (i.e., anti-KDR antibodies do not inhibit other VEGF receptors). Due to this high specificity, anti-KDR antibodies may limit and/or avoid off-target effects and toxicity caused by less specific TKIs (Witte et al., Cancer Metastasis Rev., Jun;17(2):155, 1998) .

Unlike Avastin, which binds to only one ligand (VEGF-A), anti-KDR antibodies are expected to prevent all known VEGFs from binding to VEGFR2/KDR. Anti-KDR antibodies may have a more potent inhibitory effect on tumor angiogenesis rather than simply blocking VEGF-A. There is a possibility that therapy with anti-KDR antibodies may be effective in cases of Avastin resistance.

Under this technical background, the inventors of the present application produced a novel antibody having increased affinity for an antigen by substituting VH1 in the FR (framework region) of the heavy chain variable region with VH3 and VL1 in the light chain FR (framework region) with VK1 and inducing mutations in the CDR3 regions of the heavy and light chains, in order to modify existing antibodies that specifically bind to VEGFR2/KDR, and also produced a fusion protein by fusing the DLL4 binding site of Notch1 to the terminus of the antibody, thus culminating in the present invention.

### [Disclosure]

It is an object of the present invention to provide a fusion protein including an antibody having increased binding ability to VEGFR2/KDR and a DLL4 binding protein domain fused to the terminus thereof.

It is another object of the present invention to provide a nucleic acid encoding the fusion protein.

It is still another object of the present invention to provide a vector including the nucleic acid, a transformed cell including the vector, and a method of producing the same.

It is yet another object of the present invention to provide a composition for preventing or treating an angiogenic disease including the fusion protein; to provide a method of preventing or treating an angiogenic disease, including administering the fusion protein to a subject; and to provide the use of the fusion protein for the manufacture of a composition for preventing or treating an angiogenic disease.

It is still yet another object of the present invention to provide a composition for diagnosing an angiogenic disease including the fusion protein; to provide a method of diagnosing an angiogenic disease, including administering the fusion protein to a subject or treating a sample with the fusion protein; and to provide the use of the fusion protein for the manufacture of a composition for diagnosing an angiogenic disease.

It is even still another object of the present invention to provide a composition for preventing or treating a tumor or cancer including the fusion protein; to provide a method of preventing or treating a tumor or cancer, including administering the fusion protein to a subject; and to provide the use of the fusion protein for the manufacture of a composition for preventing or treating a tumor or cancer.

It is even yet another object of the present invention to provide a composition for co-administration with an additional therapeutic agent including the fusion protein; to provide a method for co-administration with an additional therapeutic agent, including administering the fusion protein to a subject; and to provide the use of the fusion protein for the manufacture of a composition for co-administration with an additional therapeutic agent.

In order to accomplish the above objects, the present invention provides a fusion protein including an antibody binding to VEGFR2/KDR or an antigen-binding fragment thereof and a DLL4 (delta-like ligand 4) binding protein domain.

Here, the antibody binding to VEGFR2/KDR or the antigen-binding fragment thereof may include a heavy chain variable region including the heavy chain CDR1 of SEQ ID NO: 1, the heavy chain CDR2 of SEQ ID NO: 2, and the heavy chain CDR3 of SEQ ID NO: 3, and a light chain variable region including the light chain CDR1 of SEQ ID NO: 4, the light chain CDR2 of SEQ ID NO: 5, and the light chain CDR3 of SEQ ID NO: 6.

The present invention also provides a nucleic acid encoding the fusion protein.

The present invention also provides a vector including the nucleic acid.

The present invention also provides transformed cells including the vector.

The present invention also provides a method of producing the fusion protein including (a) producing a fusion protein by culturing the cells and (b) recovering the produced fusion protein.

The present invention also provides a composition for preventing or treating an angiogenic disease including the fusion protein.

The present invention also provides a composition for diagnosing an angiogenic disease including the fusion protein.

The present invention also provides a composition for preventing or treating a tumor or cancer including the fusion protein.

The present invention also provides a composition for co-administration with an additional therapeutic agent including the fusion protein.

### [Description of Drawings]

FIG. 1 shows 6A6 sequences with heavy chain CDR grafts;
FIG. 2 shows 6A6 sequences with light chain CDR grafts;
FIGs. 3a and 3b show results confirming the binding ability of the produced fusion protein by SPR;
FIG. 4 shows results confirming the ability to simultaneously bind to two antigens by SPR;
FIG. 5 shows results confirming inhibition of proliferation of HUVECs by growth regulators;
FIG. 6 shows results confirming the effect of the antibody on inhibiting VEGFR-2 phosphorylation in vascular endothelial cells;
FIG. 7 shows results confirming the binding ability of the fusion protein in vascular endothelial cells using FACS; and
FIG. 8 shows results confirming the binding ability of the fusion protein to hDLL4 expressed on the cell surface using FACS.

### [Mode for Invention]

Unless otherwise defined, all technical and scientific terms used herein have the same meanings as typically understood by those skilled in the art to which the present invention belongs. In general, the nomenclature used herein is well known in the art and is typical.

An aspect of the present invention relates to a fusion protein including an antibody binding to VEGFR2/KDR or an antigen-binding fragment thereof and a DLL4 binding protein domain, in which the antibody binding to VEGFR2/KDR or the antigen-binding fragment thereof may include a heavy chain variable region including the heavy chain CDR1 of SEQ ID NO: 1, the heavy chain CDR2 of SEQ ID NO: 2, and the heavy chain CDR3 of SEQ ID NO: 3, and a light chain variable region including the light chain CDR1 of SEQ ID NO: 4, the light chain CDR2 of SEQ ID NO: 5, and the light chain CDR3 of SEQ ID NO: 6.

In the fusion protein according to the present invention, the antibody binding to VEGFR2/KDR or the antigen-binding fragment thereof is a modified antibody of a fully humanized antibody that neutralizes the vascular endothelial growth factor receptor, and in particular, is a modified antibody of a human monoclonal antibody (Korean Patent No. 10-0883430) that neutralizes the vascular endothelial growth factor receptor.

By the inventors of the present application, an antibody that targets VEGFR-2/KDR and simultaneously has reactivity to flk-1 (VEGFR-2 homolog) derived from mice or rats was invented from a fully human antibody library and modified. The excellence of this antibody has been proven through clinical trials, but the binding ability of the antibody protein to the antigen is somewhat low and the *in vivo* half-life is short. By modifying such an antibody, it is expected to increase the efficacy as an anticancer immunotherapy agent and increase the efficacy in angiogenesis-related diseases.

In order to modify existing antibodies that specifically bind to VEGFR2/KDR, a novel antibody having increased affinity for an antigen was produced by substituting VH1 in the FR (framework region) of the heavy chain variable region with VH3 and VL1 in the light chain FR (framework region) with VK1 and inducing mutations in the CDR3 regions of the heavy and light chains. Thereby, the present inventors developed an antibody with higher binding ability and better intracellular efficacy than existing antibodies that specifically bind to VEGFR2/KDR, and ascertained functions of the antibody as a desired anticancer immunotherapy agent or a therapeutic agent for angiogenesis-related diseases.

The modified antibody was screened as follows. First, the CDR and framework (FR) sequences of 6A6 were identified, and using the CDR grafting technique, the heavy chain CDR sequence was grafted into the human germline VH3 sequence, and the light chain CDR sequence was grafted into human germline VK1.

CDR grafting is generally a method including isolating DNA encoding the variable region sequence of a mouse monoclonal antibody, obtaining the CDR sequence through gene cloning, and then grafting the CDR sequence into a suitable human antibody sequence *in silico* (Hou S, et al. Humanization of an anti-CD34 monoclonal antibody by complementarity-determining region grafting based on computer-assisted molecular modelling. J Biochem. 2 0 0 8 ; 1 4 4 (1) : 11 5 - 2 0 ; and Kashmiri SV, De Pascalis R, Gonzales NR, Schlom J. SDR grafting--a new approach to antibody humanization. Methods. 2005;36(1):25-34). Using the above method, the framework of 6A6 with human germline VH1/VL1 was substituted with human germline VH3/VK1.

In order to improve the affinity of the substituted antibody, a library that induces mutations in the CDR3 sequences of the light and heavy chains was constructed, and the antibody was screened using a recombinant KDR D1-D3-Fc fusion protein. Antibody screening was performed using phage display.

Based thereon, the present invention includes an antibody binding to VEGFR2/KDR or an antigen-binding fragment thereof, including a heavy chain variable region including the heavy chain CDR1 of SEQ ID NO: 1, the heavy chain CDR2 of SEQ ID NO: 2, and the heavy chain CDR3 of SEQ ID NO: 3, and a light chain variable region including the light chain CDR1 of SEQ ID NO: 4, the light chain CDR2 of SEQ ID NO: 5, and the light chain CDR3 of SEQ ID NO: 6.

The fusion protein of the present invention is able to specifically bind to each of VEGFR2/KDR and DLL4. "Bispecific" or "dual-specific" is the property of a binding protein able to specifically bind to two different targets and modulate the activity of the targets, and such a protein may be produced by, for example, an antibody or protein that binds specifically to each target, or fragments thereof, which possesses two distinct antigen-binding arms (specificity for two targets) and is monovalent for each antigen binding thereto.

As used herein, the term "antibody" refers to an anti-VEGFR2/KDR antibody that specifically binds to VEGFR2/KDR. The scope of the present invention includes not only the complete antibody form that specifically binds to VEGFR2/KDR, but also antigen-binding fragments of the antibody molecule.

A complete antibody has a structure having two full-length light chains and two full-length heavy chains, and each light chain is linked to a heavy chain by a disulfide bond. The heavy chain constant region has gamma (γ), mu (µ), alpha (α), delta (δ), and epsilon (ε) types, and gamma 1 (γ1), gamma 2 (γ2), gamma 3 (γ3), gamma 4 (γ4), alpha 1 (α1), and alpha 2 (α2) subclasses. The light chain constant region has kappa (κ) and lambda (λ) types.

The antigen-binding fragment of an antibody or the antibody fragment is a fragment having an antigen-binding function, and includes Fab, F(ab'), F(ab')2, and Fv. Among antibody fragments, Fab has a structure having light and heavy chain variable regions, a light chain constant region, and a first heavy chain constant region (CH1), and has one antigen-binding site. Fab' differs from Fab in that Fab' has a hinge region including at least one cysteine residue at the C-terminus of the heavy chain CH1 domain. The F(ab')2 antibody is produced by a disulfide bond between cysteine residues in the hinge region of Fab'. Fv is a minimal antibody fragment having only a heavy chain variable region and a light chain variable region. Two-chain Fv is configured such that a heavy chain variable region and a light chain variable region are linked by a non-covalent bond, and single-chain Fv (scFv) is configured such that a heavy chain variable region and a light chain variable region are generally linked by a covalent bond via a peptide linker therebetween, or are directly linked at the C-terminus, forming a dimeric structure, like the two-chain Fv. Such antibody fragments may be obtained using proteolytic enzymes (e.g., Fab may be obtained by restriction-cleaving a whole antibody with papain, and F(ab')2 may be obtained by cleavage with pepsin), or may be constructed through genetic recombination technology.

In an embodiment, the antibody according to the present invention is in Fv form (e.g., scFv) or in intact antibody form. Also, the heavy chain constant region may be any one selected from among isotypes such as gamma (γ), mu (µ), alpha (α), delta (δ), and epsilon (ε). For example, the constant region may be gamma 1 (IgG1), gamma 3 (IgG3), or gamma 4 (IgG4). The light chain constant region may be kappa or lambda type.

As used herein, the term "heavy chain" is understood to include a full-length heavy chain and fragments thereof, the full-length heavy chain including a variable region domain VH including an amino acid sequence having a variable region sequence sufficient to confer specificity to an antigen and three constant region domains CH1, CH2, and CH3. Also, the term "light chain" used herein is understood to include a full-length light chain and fragments thereof, the full-length light chain including a variable region domain VL including an amino acid sequence having a variable region sequence sufficient to confer specificity to an antigen and a constant region domain CL.

Examples of the antibody of the present invention include, but are not limited to, monoclonal antibodies, multispecific antibodies, human antibodies, humanized antibodies, chimeric antibodies, single-chain Fvs (scFVs), single-chain antibodies, Fab fragments, F(ab') fragments, disulfide-liked Fvs (sdFVs), anti-idiotype (anti-Id) antibodies, epitope-binding fragments of these antibodies, and the like.

A monoclonal antibody is an antibody obtained from a population of substantially homogeneous antibodies, in which the individual antibodies that make up the population are identical, except for possible naturally-occurring mutations that may be present at low frequency. A monoclonal antibody is highly specific and is induced against a single antigenic site. In contrast to typical (polyclonal) antibodies, which commonly include different antibodies directed against different determinants (epitopes), each monoclonal antibody is directed against a single determinant on the antigen.

The term "epitope" refers to a protein determinant to which an antibody is able to specifically bind. The epitope is usually composed of a group of chemically active surface molecules, for example amino acids or sugar side-chains, and generally has specific three-dimensional structural features and specific charge properties. Conformational and nonconformational epitopes are distinguished in that binding to the former, but not the latter, is lost in the presence of a denaturing solvent.

A non-human (e.g., murine) antibody in a "humanized" form is a chimeric antibody that contains a minimal sequence derived from a non-human immunoglobulin. In most cases, the humanized antibody is a human immunoglobulin (recipient antibody), in which a residue from the hypervariable region of a recipient is replaced with a residue from the hypervariable region of a non-human species (donor antibody) having the desired specificity, affinity, and capability, for example, mice, rats, rabbits, or non-human primates.

A "human antibody" is a molecule derived from human immunoglobulin, and means that all of the amino acid sequences constituting the antibody including a complementarity-determining region and a framework region are composed of human immunoglobulin.

A portion of the heavy chain and/or light chain is identical to or homologous with the corresponding sequence in an antibody derived from a particular species or belonging to a particular antibody class or subclass, whereas the remaining chain(s) includes a "chimeric" antibody (immunoglobulin) that is identical to or homologous with the corresponding sequence in an antibody derived from another species or belonging to another antibody class or subclass, as well as fragments of such antibodies that exhibit the desired biological activity.

The "variable region" of the antibody as used herein is a light chain or heavy chain portion of an antibody molecule including the amino acid sequence of a complementarity-determining region (CDR; i.e., CDR1, CDR2, and CDR3) and a framework region (FR). VH refers to the variable region of a heavy chain, and VL refers to the variable region of a light chain.

The "complementarity-determining region" (CDR; i.e., CDR1, CDR2, and CDR3) is an amino acid residue of the antibody variable region that is necessary for antigen binding. Each variable region typically has three CDRs, identified as CDR1, CDR2, and CDR3. The present invention includes a heavy chain variable region including the heavy chain CDR3 of SEQ ID NO: 3 and a light chain variable region including the light chain CDR3 of SEQ ID NO: 6.

In the present invention, the antibody binding to VEGFR2/KDR and DLL4 or the antigen-binding fragment thereof may include a heavy chain variable region including the heavy chain CDR1 of SEQ ID NO: 1, the heavy chain CDR2 of SEQ ID NO: 2, and the heavy chain CDR3 of SEQ ID NO: 3, and a light chain variable region including the light chain CDR1 of SEQ ID NO: 4, the light chain CDR2 of SEQ ID NO: 5, and the light chain CDR3 of SEQ ID NO: 6.

The "framework region" (FR) is a variable region residue other than the CDR residue. Each variable region typically has four FRs, identified as FR1, FR2, FR3, and FR4.

The "Fv" fragment is an antibody fragment that contains a complete antibody recognition and binding site. This region is composed of a dimer of one heavy chain variable region and one light chain variable region tightly covalently associated, for example, with scFv.

The "Fab" fragment contains the variable and constant domains of the light chain and the variable and first constant domains (CH1) of the heavy chain. The F(ab')2 antibody fragment generally includes a pair of Fab fragments covalently linked near the carboxy termini thereof by a hinge cysteine therebetween.

A "single-chain Fv" or "scFv" antibody fragment includes the VH and VL domains of an antibody, and these domains are present within a single polypeptide chain. The Fv polypeptide may further include a polypeptide linker between the VH and VL domains to enable scFv to form the desired structure for antigen binding.

The VEGFR2/KDR antibody may include a single chain or two chains. Functionally, binding affinity of the VEGFR2/KDR antibody falls in the range of 10⁻⁵ M to 10⁻¹² M. For example, the binding affinity of the VEGFR2/KDR antibody may be 10⁻⁶ M to 10⁻¹² M, 10⁻⁷ M to 10-¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻³ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M.

The present invention includes an antibody binding to VEGFR2/KDR or an antigen-binding fragment thereof, including a heavy chain variable region including a sequence with at least 80% homology to the sequence of SEQ ID NO: 8.

The present invention also includes an antibody binding to VEGFR2/KDR or an antigen-binding fragment thereof, including a light chain variable region including a sequence with at least 80% homology to the sequence of SEQ ID NO: 10.

The present invention also includes an antibody binding to VEGFR2/KDR or an antigen-binding fragment thereof, including a heavy chain variable region including a sequence having at least 80% homology to the sequence of SEQ ID NO: 8 and a light chain variable region including a sequence having at least 80% homology to the sequence of SEQ ID NO: 10.

The homology may be 80%, 81%, 82%, 83%, 84%, 85%, 86%, 87%, 88%, 89%, 90%, 91%, 92%, 93%, 94%, 95%, 96%, 97%, 98%, 99%, or more, compared to the entire sequence of the claimed sequence number.

The present invention may include not only the sequence of the fusion protein set forth therein, but also biological equivalents thereto. For example, additional modifications may be made to the amino acid sequence in order to further improve the binding affinity and/or other biological properties of the fusion protein. Such modifications include, for example, deletion, insertion, and/or substitution of the amino acid sequence residues. The amino acid variations are based on the relative similarity of amino acid side-chain substituents with regard to, for example, hydrophobicity, hydrophilicity, charge, size, and the like. Based on analysis of the size, shape, and type of amino acid side-chain substituents, all of arginine, lysine, and histidine are positively charged residues, alanine, glycine, and serine have similar sizes, and phenylalanine, tryptophan, and tyrosine have similar shapes. Therefore, based on these considerations, arginine, lysine, and histidine may be regarded as biologically functional equivalents, alanine, glycine, and serine may be regarded as biologically functional equivalents, and phenylalanine, tryptophan, and tyrosine may be regarded as biologically functional equivalents.

Taking into consideration the above-described variations having equivalent biological activity, the fusion protein of the present invention or the nucleic acid molecule encoding the same is to be understood as including a sequence showing substantial identity to the sequence set forth in the sequence number. When the sequence of the present invention and any other sequences are aligned so as to correspond to each other as closely as possible and the aligned sequence is analyzed using an algorithm commonly used in the art, "substantial identity" refers to sequences exhibiting at least 90% homology, most preferably at least 95% homology, at least 96% homology, at least 97% homology, at least 98% homology, or at least 99% homology. Alignment methods for sequence comparison are known in the art. The NCBI Basic Local Alignment Search Tool (BLAST) is accessible through NCBI and elsewhere, and may be used in conjunction with sequencing programs such as blastp, blasm, blastx, tblastn, and tblastx on the Internet. BLAST is available at www.ncbi.nlm.nih.gov/BLAST/. A method for comparing sequence homology using this program may be found at www.ncbi.nlm.nih.gov/BLAST/blast_help.html.

Based thereon, the fusion protein of the present invention may have 90%, 91%, 92%, 93%, 94%, 95%, 96%, 97%, 98%, 99%, or higher homology with a specified sequence or all of the sequences set forth herein. Such homology may be determined through sequence comparison and/or alignment using methods known in the art. For example, the percentage sequence homology of a nucleic acid or protein of the present invention may be determined using a sequence comparison algorithm (i.e., BLAST or BLAST 2.0), manual alignment, or visual inspection.

The DLL4 binding protein domain is a protein domain that binds to DLL4 and includes the DLL4 binding site of Notch1. For example, it may include the EGF-like domain of human Notch1 and may include the sequence of SEQ ID NO: 18.

The DLL4 binding protein domain may bind to the terminus of an antibody that binds to VEGFR2/KDR, for example, to the terminus of a light chain, particularly to the N-terminus of a light chain.

Korean Patent No. 10-1569083 discloses a DIG-KN material including VEGFR2/KDR and a DLL4 binding protein domain, but the present invention differs therefrom in that there is provided an anti-VEGFR2 (KDR) antibody having increased affinity for an antigen by substituting VH1 in the heavy chain FR (framework region) of a human antibody that specifically binds to VEGFR2/KDR with VH3 and VL1 in the light chain FR (framework region) with VK1 and inducing mutations therein and also that the DLL4 binding protein domain (Notch1 EGF-like domain) binds to the N-terminus of the light chain of the anti-VEGFR2 (KDR) antibody.

The antibody binding to VEGFR2/KDR or the antigen-binding fragment thereof and the DLL4 binding protein domain may be connected through a linker. The linker may be a peptide linker and may have a length of about 10-25 aa. For example, hydrophilic amino acids such as glycine and/or serine may be included, but the present invention is not limited thereto.

Specifically, the linker may include, for example, (GS)n, (GGS)n, (GSGGS)n, or (GnS)m (in which n and m are each from 1 to 10), but the linker may be, for example, (GnS)m (in which n and m are each from 1 to 10).

Another aspect of the present invention relates to a nucleic acid encoding the fusion protein.

The nucleic acid may include the sequence of SEQ ID NO: 7 encoding the heavy chain variable region. The nucleic acid may include the sequence of SEQ ID NO: 9 encoding the light chain variable region. The nucleic acid may include the sequence of SEQ ID NO: 17 encoding the DLL4 binding protein domain.

The nucleic acid encoding the fusion protein of the present invention may be isolated and produced recombinantly. The nucleic acid may be isolated and inserted into a replicable vector for further cloning (DNA amplification) or further expression. Based thereon, still another aspect of the present invention relates to a vector including the nucleic acid.

Here, "nucleic acid" has a meaning comprehensively encompassing DNA (gDNA and cDNA) and RNA molecules, and nucleotides, which are the basic building blocks of nucleic acids, include not only natural nucleotides but also analogues in which sugar or base regions are modified. The sequence of the nucleic acid encoding the fusion protein of the present invention may be modified. Such modification includes addition, deletion, or non-conservative or conservative substitution of nucleotides.

DNA is easily isolated or synthesized using typical techniques (e.g., using an oligonucleotide probe capable of specifically binding to DNA encoding the heavy and light chains of the antibody). Many vectors are available. A vector component generally includes, but is not limited to, at least one selected from among a signal sequence, an origin of replication, at least one marker gene, an enhancer element, a promoter, and a transcription termination sequence.

As used herein, the term "vector" refers to a means for expressing a target gene in a host cell, and includes a plasmid vector, a cosmid vector, a virus vector, such as a bacteriophage vector, an adenovirus vector, a retrovirus vector, and an adeno-associated virus vector, etc. In the vector, the nucleic acid encoding the fusion protein is operably linked with a promoter.

Here, "operably linked" means a functional linkage between a nucleic acid expression control sequence (e.g., a promoter, a signal sequence, or an array of transcription regulator binding sites) and a different nucleic acid sequence, whereby the control sequence serves to control the transcription and/or translation of the different nucleic acid sequence.

When a prokaryotic cell is used as a host, a strong promoter capable of promoting transcription (e.g., a tac promoter, lac promoter, lacUV5 promoter, lpp promoter, pLλ promoter, pRX promoter, rac5 promoter, amp promoter, recA promoter, SP6 promoter, trp promoter, or T7 promoter), a ribosome-binding site for initiation of translation, and a transcription/translation termination sequence are generally included. In addition, for example, when a eukaryotic cell is used as a host, a promoter derived from the genome of a mammalian cell (e.g., a metallothionine promoter, β-actin promoter, human hemoglobin promoter, or human muscle creatine promoter) or a promoter derived from a mammalian virus (e.g., an adenovirus late promoter, vaccinia virus 7.5k promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), or promoter of Rous sarcoma virus (RSV)) may be used, and a polyadenylation sequence is generally used as a transcription termination sequence.

In some cases, the vector may be fused with another sequence in order to facilitate purification of the fusion protein expressed therefrom. Examples of the sequence that is fused therewith include glutathione S-transferase (Pharmacia, USA), maltose-binding protein (NEB, USA), FLAG (IBI, USA), and 6x His (hexa-histidine; Qiagen, USA).

The vector contains, as a selective marker, an antibiotic resistance gene that is commonly used in the art, for example, a gene conferring resistance to ampicillin, gentamicin, carbenicillin, chloramphenicol, streptomycin, kanamycin, geneticin, neomycin, or tetracycline.

Yet another aspect of the present invention relates to cells transformed with the vector described above. Examples of the cells used to produce the fusion protein of the present invention may include, but are not limited to, prokaryotic cells, yeast cells, and higher eukaryotic cells.

Prokaryotic host cells, such as *Escherichia coli, Bacillus subtilis* and *Bacillus thuringiensis* as strains belonging to the genus *Bacillus, Streptomyces, Pseudomonas* (e.g., *Pseudomonas putida*), *Proteus mirabilis,* and *Staphylococcus* (e.g., *Staphylococcus carnosus*), may be used.

Here, animal cells are of greatest interest, and examples of useful host cell lines may include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

Still yet another aspect of the present invention relates to a method of producing the fusion protein including (a) culturing the cells described above and (b) recovering the fusion protein from the cultured cells.

The cells may be cultured in various media. Any commercially available medium may be used as a culture medium without limitation. All other essential supplements known to those skilled in the art may be contained in appropriate concentrations. Culture conditions, such as temperature, pH, etc., are already in use with the host cells selected for expression, as will be apparent to those skilled in the art.

For recovery of the fusion protein, impurities may be removed by, for example, centrifugation or ultrafiltration, and the resultant product may be purified using, for example, affinity chromatography, etc. Other additional purification techniques, such as anion or cation exchange chromatography, hydrophobic interaction chromatography, hydroxyapatite chromatography, and the like, may be used.

The antibody included in the fusion protein may be IgG or a fragment including a variable region, such as scFv or Fab. Also, the variable region of the heavy chain may be IgG1, IgG2, IgG3, or IgG4.

Even still another aspect of the present invention relates to a composition for preventing or treating an angiogenic disease including the fusion protein as an active ingredient.

Here, "angiogenesis" refers to the formation or growth of new blood vessels from preexisting blood vessels, and "angiogenesis-related disease" refers to a disease related to the occurrence or progression of angiogenesis. The disease may fall within the scope of angiogenesis-related diseases without limitation so long as it may be treated with the fusion protein. Examples of the angiogenesis-related disease may include, but are not limited to, cancer, metastasis, diabetic retinopathy, retinopathy of prematurity, corneal graft rejection, macular degeneration, neovascular glaucoma, erythrosis, proliferative retinopathy, psoriasis, hemophilic arthritis, capillary formation of atherosclerotic plaques, keloid, wound granulation, vascular adhesion, rheumatoid arthritis, osteoarthritis, autoimmune diseases, Crohn's disease, restenosis, atherosclerosis, intestinal adhesions, cat scratch disease, ulcer, liver cirrhosis, nephritis, diabetic nephropathy, diabetic foot ulcers, chronic kidney failure, diabetes mellitus, inflammatory diseases, idiopathic pulmonary fibrosis, sepsis, acute respiratory distress syndrome, and neurodegenerative diseases.

Moreover, the cancer may be selected from the group consisting of esophageal cancer, stomach cancer, large intestine cancer, rectal cancer, oral cancer, pharynx cancer, larynx cancer, lung cancer, colon cancer, breast cancer, uterine cervical cancer, endometrial cancer, ovarian cancer, prostate cancer, testicular cancer, bladder cancer, renal cancer, liver cancer, pancreatic cancer, bone cancer, connective tissue cancer, skin cancer, brain cancer, thyroid cancer, leukemia, Hodgkin's lymphoma, lymphoma, and multiple myeloid blood cancer, but is not limited thereto.

As used herein, the term "prevention or prophylaxis" refers to any action that inhibits or delays the onset of a disease of interest by administering the fusion protein of the present invention. The term "treatment or therapy" refers to any action that ameliorates or alleviates symptoms of a disease of interest by administering the fusion protein of the present invention.

The present invention may be a pharmaceutical composition for preventing or treating a tumor or cancer, including, for example, (a) a pharmaceutically effective amount of the fusion protein according to the present invention and (b) a pharmaceutically acceptable carrier. The present invention may also be a method of preventing or treating a tumor or cancer, including administering the fusion protein to a patient suffering from a tumor or cancer. Furthermore, the present invention may be the use of the fusion protein for interfering with the mechanism and for preventing or treating a tumor or cancer thereby.

Non-limiting examples of the tumor or cancer preferred for treatment include melanoma (e.g., metastatic malignant melanoma), kidney cancer (e.g., clear cell carcinoma), prostate cancer (e.g., hormone-refractory prostatic adenocarcinoma), pancreatic adenocarcinoma, breast cancer, colon cancer, lung cancer (e.g., non-small cell lung cancer), esophageal cancer, head and neck squamous cell carcinoma, liver cancer, ovarian cancer, uterine cervical cancer, thyroid cancer, glioblastoma, glioma, leukemia, lymphoma, and other neoplastic carcinomas. Additionally, the present invention includes refractory or relapsed cancer that may be treated using the fusion protein.

The present invention provides, for example, a method of treating an angiogenic disease, including administering a therapeutically effective amount of the fusion protein to a patient in need of angiogenesis inhibition. The treatment of the angiogenic disease may further include identifying a patient in need of angiogenesis inhibition before the administration step. In another example, a method of preventing and/or treating an angiogenic disease is provided, including administering a therapeutically effective amount of the fusion protein to a patient. The prevention and/or treatment method may further include identifying a patient in need of prevention and/or treatment of an angiogenic disease before the administration step.

The pharmaceutical composition may further include a pharmaceutically acceptable carrier, and the carrier is commonly used in formulation of drugs and may be at least one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but is not limited thereto. The pharmaceutical composition may further include at least one selected from the group consisting of diluents, excipients, lubricants, wetting agents, sweeteners, flavoring agents, emulsifiers, suspending agents, and preservatives commonly used in preparation of pharmaceutical compositions.

The pharmaceutical composition, or an effective amount for the angiogenic disease, may be administered orally or parenterally. Parenteral administration may include intravenous injection, subcutaneous injection, intramuscular injection, intraperitoneal injection, intradermal administration, topical administration, intranasal administration, intrapulmonary administration, intrarectal administration, etc. When administered orally, proteins or peptides may be digested, so oral compositions may be formulated to coat the active agent or protect the same from degradation in the stomach. Moreover, the composition may be administered by any device capable of transporting the active material to target cells.

The composition of the present invention is administered in a pharmaceutically effective amount. As used herein, the term "pharmaceutically effective amount" refers to an amount of a pharmaceutical composition sufficient to treat a disease at a reasonable benefit/risk ratio applicable to any medical treatment. The effective amount varies depending on various factors including severity of the disease to be treated, the patient's age and gender, type of disease, activity of the drug, sensitivity to the drug, administration time, administration route, secretion rate, treatment period, co-administration of the drug, and other parameters known in the art. The composition of the present invention may be administered alone or in combination with other therapeutics. As such, the composition may be administered sequentially or simultaneously with conventional therapeutics. Also, the composition may be administered in a single dose or multiple doses. Taking these factors into full consideration, it is important to administer the minimum amount sufficient to obtain a maximum effect without side effects, and this dose may be readily determined by an expert in the field. The dose of the pharmaceutical composition of the present invention is not particularly limited, but varies depending on various factors, including the patient's health status and body weight, severity of the disease, type of drug, administration route, and administration time. The composition may be administered in a single dose or multiple doses per day to mammals, including rats, mice, livestock, humans, etc., by typically accepted routes, for example, orally, intrarectally, intravenously, subcutaneously, intrauterinely, or intracerebrovascularly.

The content of the fusion protein in the pharmaceutical composition may be variously set depending on factors such as formulation method, administration mode, patient's age, body weight, gender, pathological condition, food, administration time, administration interval, administration route, excretion rate, and response sensitivity. For example, the daily dosage of the fusion protein may be in the range of 0.001 to 1000 mg/kg, particularly 0.01 to 100 mg/kg, more particularly 0.1 to 50 mg/kg, even more particularly 0.1 to 20 mg/kg, but is not limited thereto. The daily dosage may be formulated as a single preparation in unit dose form, may be formulated in appropriate portions, or may be prepared by placing the same in a multi-dose container.

The pharmaceutical composition may be administered in combination with other drugs, such as other therapeutic agents for diseases related to angiogenesis inhibitors, and the dosage, administration method, and type of other drugs may be appropriately set depending on the patient's condition.

The pharmaceutical composition may be in the form of a solution, suspension, syrup or emulsion in oil or aqueous medium, or may be formulated in the form of extracts, powders, powders, granules, tablets or capsules, and may further include a dispersant or stabilizer for formulation.

In particular, the pharmaceutical composition including the fusion protein may be formulated into an immune liposome. A liposome including the fusion protein may be prepared according to methods well known in the art. The immune liposome is a lipid composition including phosphatidylcholine, cholesterol, and polyethylene glycol-derivatized phosphatidylethanolamine and may be prepared by reverse phase evaporation. (Korean Patent Application Publication No. 10-2015-0089329). For example, the Fab' fragment of an antibody may be conjugated to the liposome by disulfide replacement reaction.

Also, the fusion protein of the present invention may be used in combination with other agents, antibodies, biologically active agents, or materials for various purposes. Even yet another aspect of the present invention relates to a composition for co-administration with an additional therapeutic agent for an angiogenic disease, including the fusion protein.

Examples of the additional therapeutic agent for an angiogenic disease may include anti-angiogenic drugs, anti-inflammatory drugs, and/or anticancer drugs. Thereby, these may overcome each other's resistance and improve efficacy.

When the composition according to the present invention is co-administered with an additional therapeutic agent for an angiogenic disease, the fusion protein and the additional therapeutic agent for an angiogenic disease may be administered sequentially or simultaneously. For example, an anti-angiogenic drug, an anti-inflammatory drug, and/or an anticancer drug may be administered to a subject and then the composition including the fusion protein as an active ingredient may be administered thereto, or the composition may be administered to a subject and then an anti-angiogenic drug, an anti-inflammatory drug, and/or an anticancer drug may be administered thereto. In some cases, the composition may be administered to a subject simultaneously with an anti-angiogenic drug, an anti-inflammatory drug, and/or an anticancer drug.

### Examples

A better understanding of the present invention may be obtained through the following examples. These examples are merely set forth to illustrate the present invention, and are not to be construed as limiting the scope of the present invention, as will be apparent to those of ordinary skill in the art.

### Example 1. CDR grafting

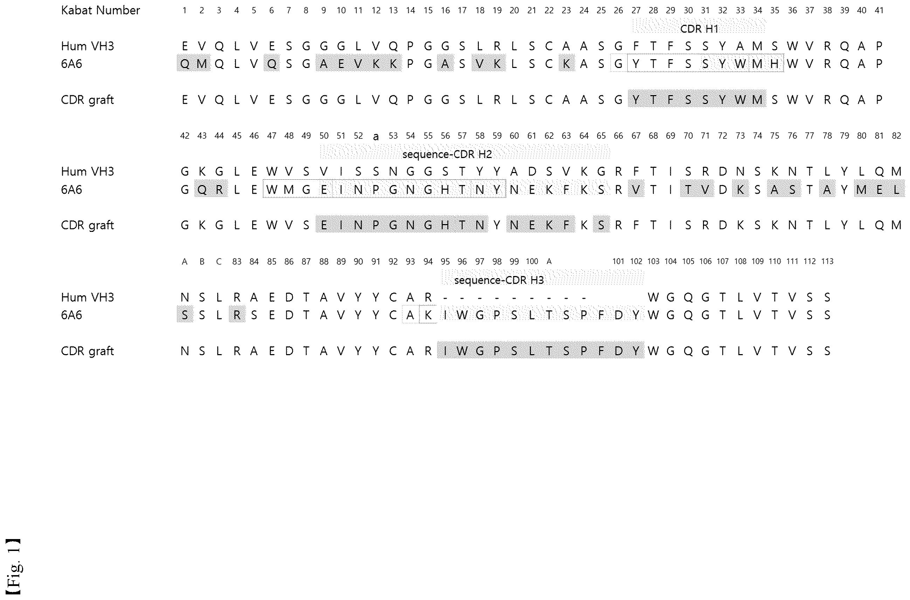

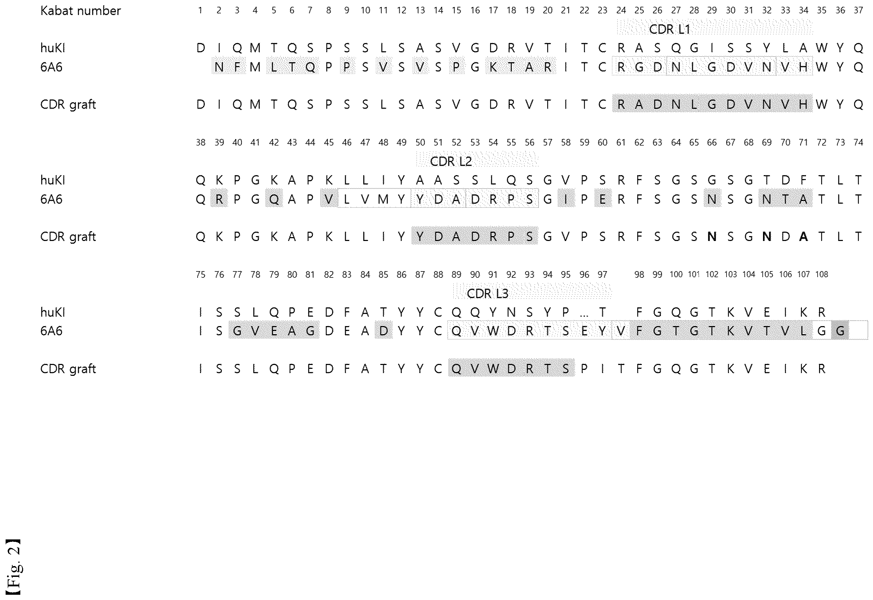

The CDR sequences of 6A6 were identified by Kabat numbering using the IMGT system, and cloning was performed to graft the same into Human germline VH3/VK1. The heavy chain CDR grafts are shown in FIG. 1, and the light chain CDR grafts are shown in FIG. 2.

### Example 2. Production and selection of variants to improve affinity

Optimization was performed to increase affinity of the CDR grafted antibody. Using a soft-randomization method of randomizing the original DNA sequences of the CDR3 of the light and heavy chains with 70% conservation, primers in which random variations were introduced into light chain CDR3 and heavy chain CDR3 were constructed. By PCR using such primers, DNA fragments encoding the light and heavy chain variable regions into which variations were introduced were obtained. These DNA fragments were substituted with the light chain variable region of the scFv phage phagemid and the heavy chain variable region of the scFv phage phagemid, constructing a light chain CDR3 variant scFv phage library and a heavy chain CDR3 variant scFv phage DNA library, respectively.

The variant scFv phage DNA library was purified with phenol-chloroform and then transformed into *E. coli* strain XL-1 Blue using electroporation. After confirming that diversity was obtained by transformation efficiency analysis and DNA sequence analysis, phage expression was induced by culture on a 500 ml scale, and CDR3 variant scFv phage libraries of light and heavy chains were constructed using PEG-precipitation.

Biopanning was performed using each variant scFv phage library. 100 µl of an antigen (KDR1-3 domain-Fc) was added at 2 µg/ml to each well in a 96-well immune plate and incubated overnight at 4°C. The next day, the antigen coating plate was washed three times with PBST (0.1% Tween20), after which 200 ul of 2% BSA blocking buffer was added, followed by reaction at room temperature for 2 hours. 50 µl of XL1-Blue stock was added to 2 ml of 2x YT-TET (tetracycline 10 µg/ml) growth medium and allowed to grow at 37°C and 200 rpm for about 2 hours, followed by further addition of 13 ml thereof and growth until an OD₆₀₀ reached 0.5. After 2 hours of blocking, washing was performed three times with 1X PBST (0.1% Tween20). The variant library and 4% BSA were mixed in equal amounts and then 200 ul thereof was added to each washed well, followed by rocking at room temperature for 30 minutes and then reaction for 2 hours. After completion of phage library reaction, the supernatant was discarded, followed by washing five times with 0.1% PBST and five times with PBS. 100 ul of 100 mM TEA (trimethylamine) was added to each well and shaken at room temperature for 10 minutes. After 10 minutes, 50 µl of 1 M Tris (pH 7.5) was added to each well and mixed. The supernatant was added to 10 ml of XL1-Blue with an OD₆₀₀ of 0.5, followed by infection at 37°C for 30 minutes. After completion of infection, 100 µl thereof was used as an output titer, and the remainder was centrifuged at 6,000 rpm for 10 minutes. The supernatant was discarded, and the precipitate was spread on a large square plate (CM 34 µg/ml + 1% glucose) and incubated overnight at 37°C. The 100 ul stored as the output titer was diluted 1/10, 1/100, and 1/1000, spread on a CM plate, and incubated overnight at 37°C. The next day, colonies grown on the square plate were collected using a loop after addition of 50 ml of 2x YT medium, followed by centrifugation at 6000 rpm for 10 minutes, and the supernatant was discarded and the precipitate was made into the first panning stock. 100 ml of 2x YT culture medium (growth media: CM 34 µg/ml + 1% glucose) was placed in a 500 ml Erlenmeyer flask, and the cells were added to an OD₆₀₀ of 0.2 and allowed to grow at 200 rpm and 37°C until an OD₆₀₀ reached 0.5. After culture of the cells until an OD₆₀₀ reached 0.5, helper phage (M13KO7 mutant) was added in an amount corresponding to 20 times the amount of cells. After addition of helper phage, the cells were infected at 37°C for 30 minutes and then centrifuged at 6000 rpm for 10 minutes. The supernatant was discarded, the medium was replaced with 100 ml of 2x YT medium (CM 34 µg/ml + Kan. 70 µg/ml + 1 mM IPTG + 5 mM MgCl₂), and the cells were incubated overnight at 200 rpm and 30°C. The next day, the grown cells were centrifuged at 7000 rpm for 10 minutes and centrifuged once more in the same manner. The collected supernatant was precipitated for 1 hour on ice by addition of 20% PEG/2.5 M NaCl in an amount of 1/5 (v/v) of the supernatant. After precipitation, centrifugation was performed at 9000 rpm for 1 hour. The supernatant was discarded, and the precipitate was resuspended in 5 ml of PBS, filtered with a 0.45 um filter, and stored at 4°C for use in the next panning process. This process was repeated three or four times, and antibodies binding to the antigen were identified by ELISA.

Thereafter, in the selection process, the dissociation rate constant K_{dis} of scFv was measured as a quantitative evaluation index of the ability to maintain binding.

### Example 3. Selection of antibody with high affinity for antigen (off-rate screening)

The binding ability of the selected antibodies to the antigen was measured using Octet (Fortebio). To this end, KDR1-3 domains were immobilized on a biosensor, candidate antibodies expressed in scFv form were added and bound, and the dissociation rate constant was measured. The results of measurement of dissociation rate constant for the five selected optimized clones [Table 1] and amino acid sequences [Tables 2 and 3] are shown below.

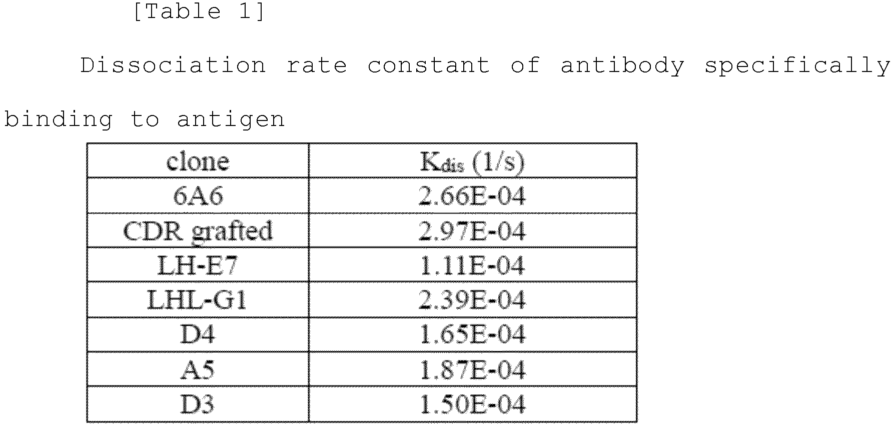

### Example 4. Production of fusion protein

To produce a fusion protein, D4, which had the lowest dissociation constant among the scFv phage antibodies selected, was converted into IgG form. Conversion into IgG form was performed using molecular biological techniques. Phagemid was extracted from the selected *E. coli* clone, and the variable regions were amplified using PCR. The amplified heavy chain variable region was inserted into an expression vector (Invivogen, pfusess-hchg1) including the heavy chain constant region, and the amplified light chain variable region was inserted into an expression vector (Invivogen, pfuse2ss-hclk) including the light chain constant region, and thus DNA cloning of IgG form was completed, resulting in conversion into IgG1 form. A DLL4 binding domain (Notch1 EGF-like domain) including a linker (G4S sequence) was fused to the N-terminus of the light chain constant region converted above by gene synthesis. The base sequences of the produced antibodies are shown in Tables 2 and 3 below.

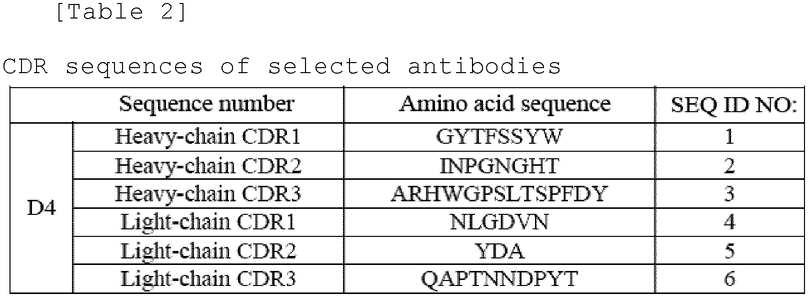

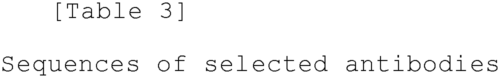

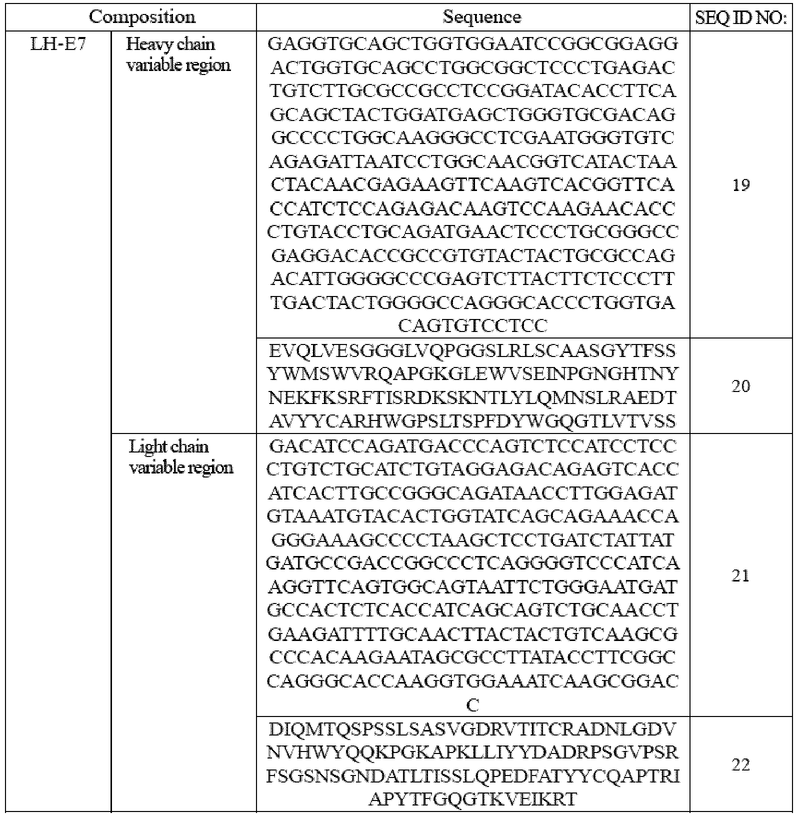

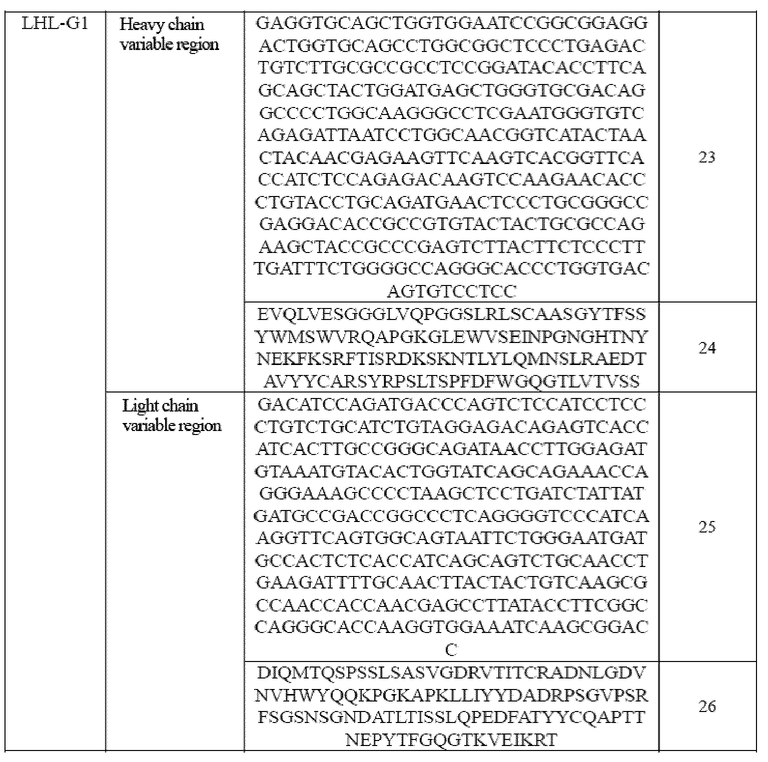

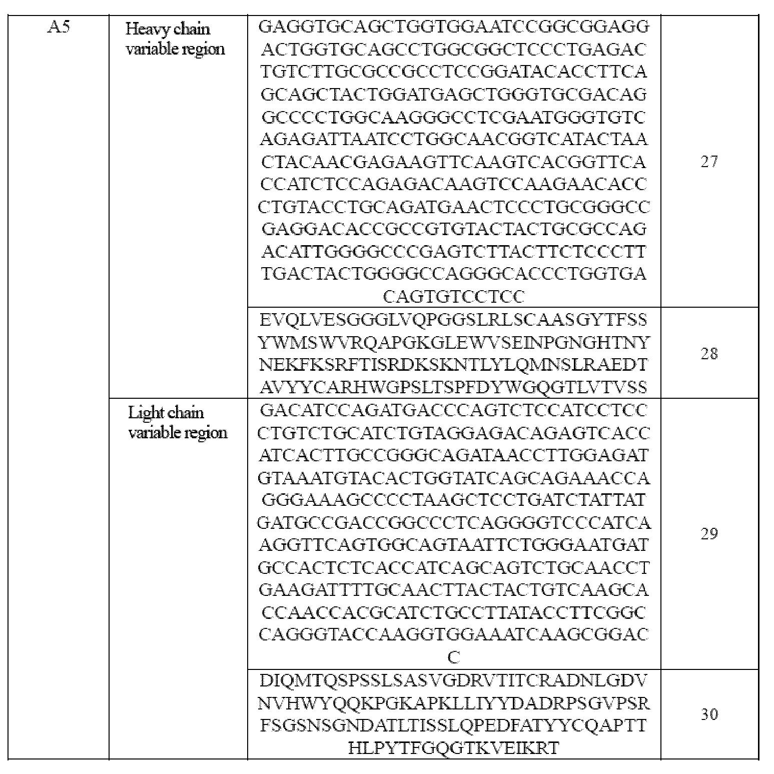

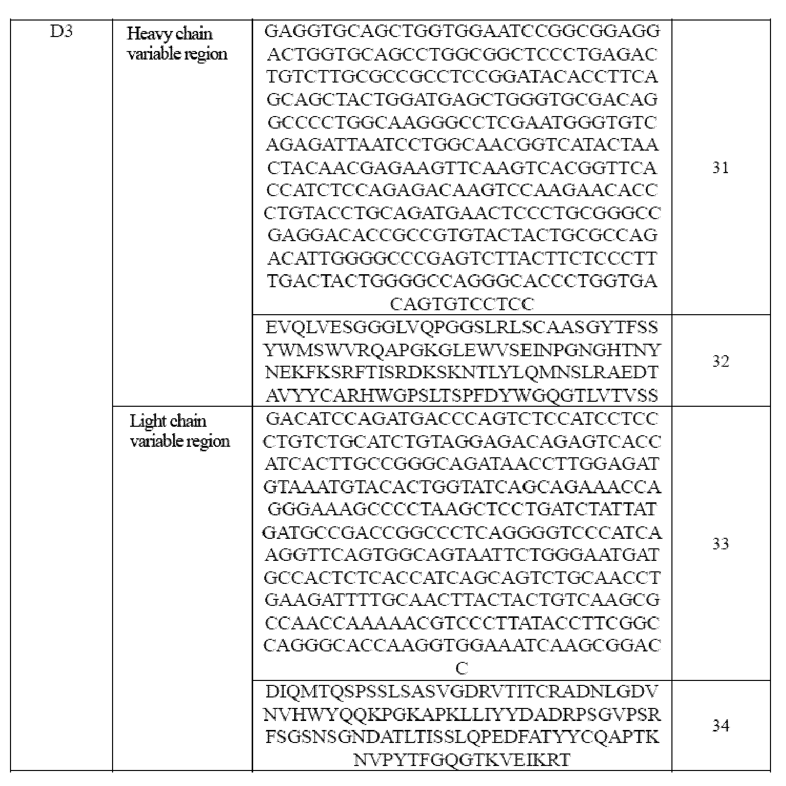

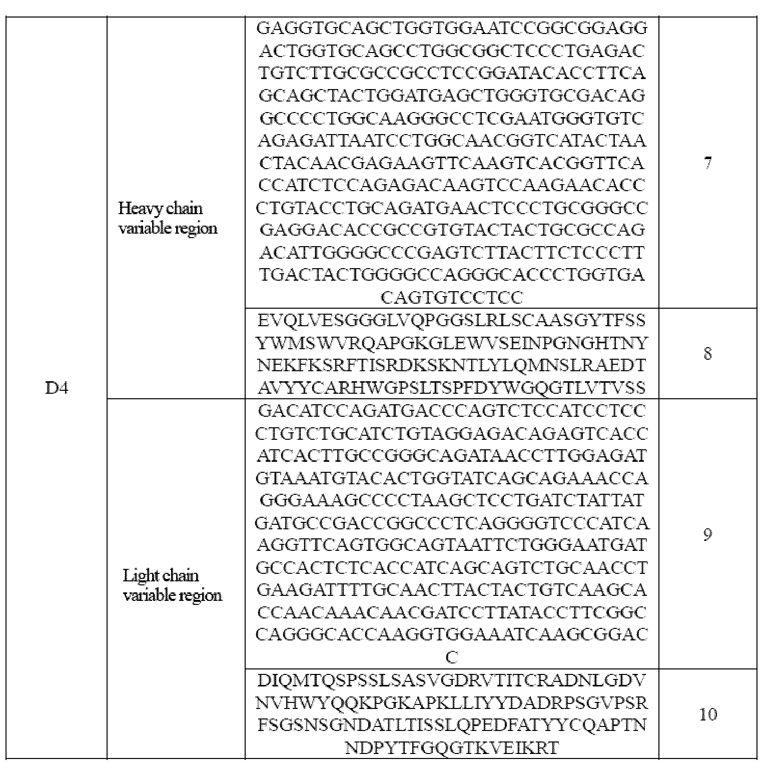

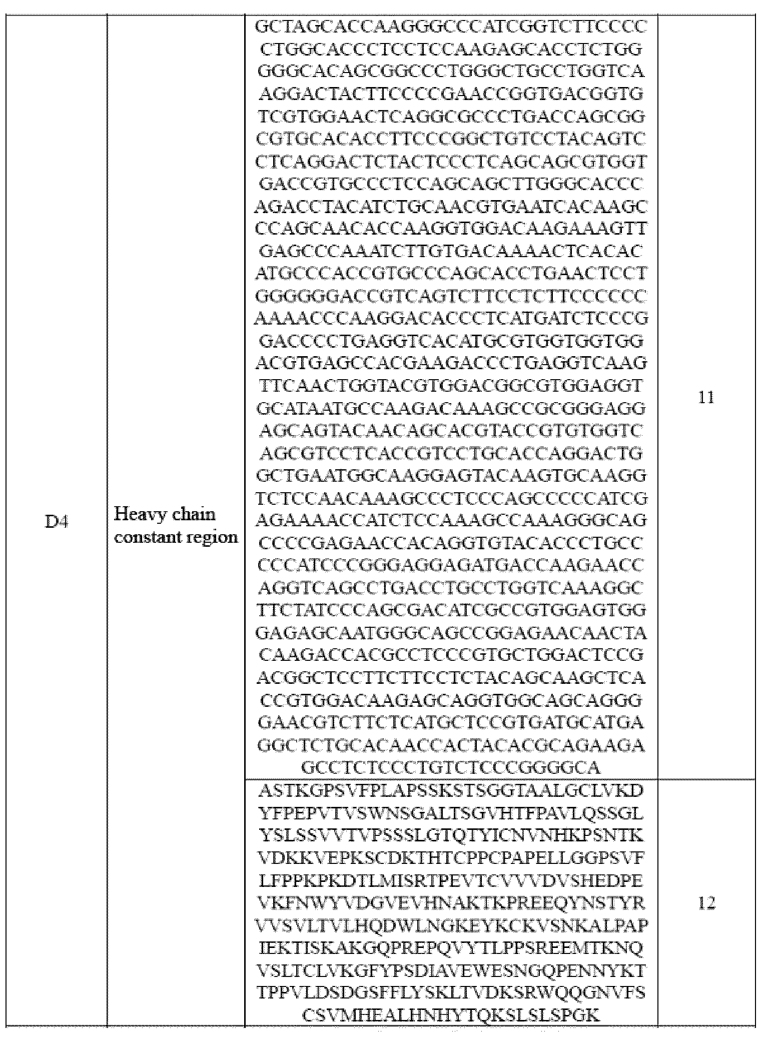

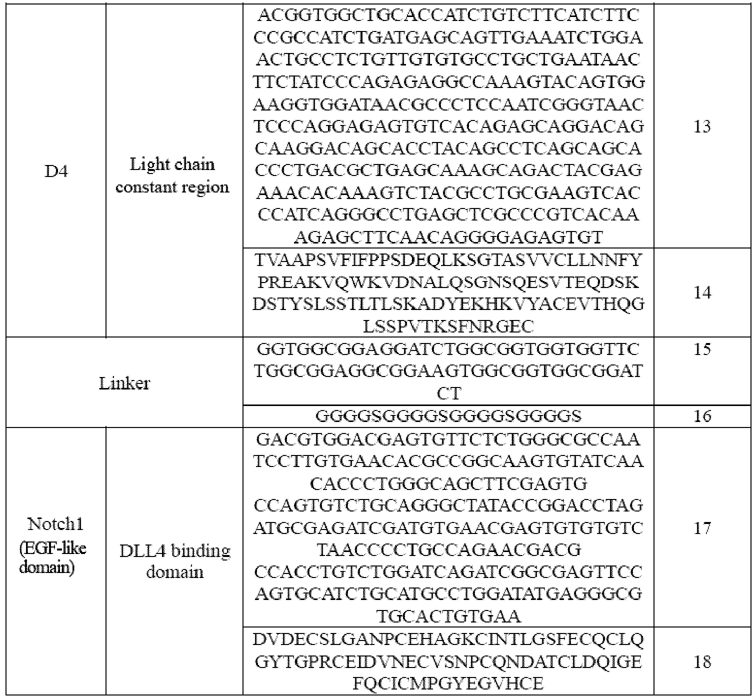

### Example 5. Expression of fusion protein

Transient expression of IgG form was performed using an Expi293F expression system kit (Thermo Fisher Scientific, US). Expi293 cells included in the kit were cultured in suspension in an orbital shaker at 125 rpm in a 5% CO₂ environment at 37°C using dedicated medium. Subculture was performed every 3 days to reach 3×10⁵ cells/ml, and when introducing the expression vector, the number of cells was adjusted to 3×10⁶ cells/ml before use. For gene introduction, a dedicated reagent, ExpiFectamine, was used, and a lipid-DNA complex containing 1 µg of expression vector DNA and 2.7 pl of ExpiFectamine per 1 ml of the cell suspension was constructed and added to the cell suspension. 16-18 hours after introduction, expression was induced by adding 1/2 of enhancer. After culture for 3-4 days under the same conditions, the supernatant containing IgG was obtained by centrifugation.

### Example 6. Purification of fusion protein

The supernatant thus obtained was injected onto a Protein A column (GE Healthcare), and IgG was purified by affinity chromatography. After equilibrating the column with 20 mM Tris-HCl, 50 mM NaCl, and 5 mM EDTA (pH 7.0), the supernatant was injected, followed by washing with 50 mM Tris-HCl, 500 mM NaCl, 5 mM EDTA, 0.2% polysorbate 20 (pH 7.0) solution, elution with 50 mM NaCl and 0.1 M glycine-HCl (pH 3.5), and then neutralization with 1 M Tris. The eluted protein was dialyzed using an MWCO 10,000 Spectra Por dialysis membrane (Spectrum Labs, US) to thus replace the solvent with PBS. Then, the protein was concentrated to a necessary concentration using Vivaspin (Satorius, DE), dispensed, and then stored at -80°C.

### Example 7. Analysis of binding specificity of fusion protein

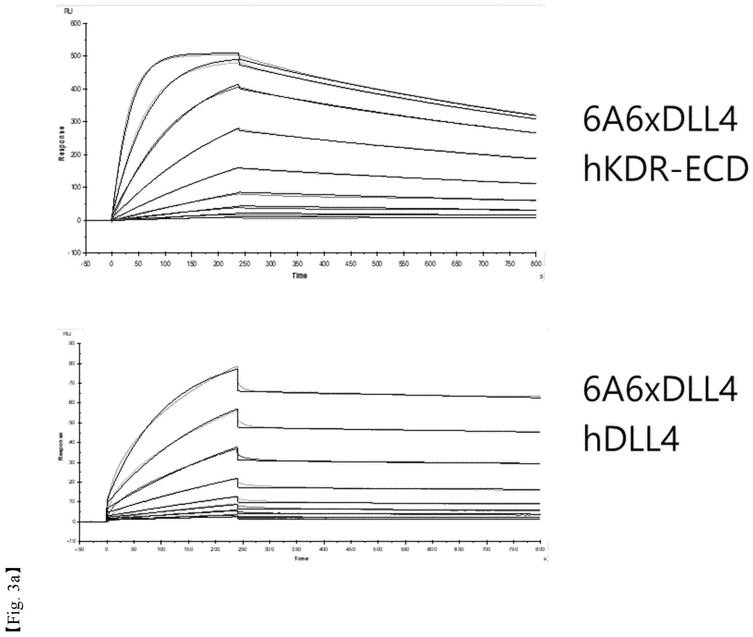

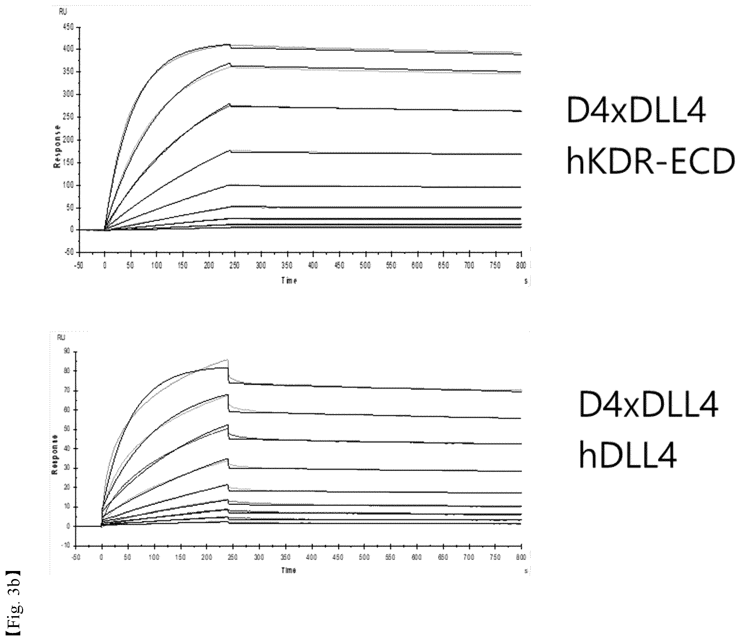

The binding ability of the selected fusion protein to the antigen was measured using SPR (Biacore T200, Cytiva). The fusion protein was captured and analyzed using the antigen protein as an analyte. Using a Protein A chip, the fusion protein was immobilized on the surface according to the manufacturer's instructions. The fusion protein on the sensor chip was diluted with PBS-T running buffer to a concentration of 5 µg/ml and injected at a flow rate of 10 µl/min to reach about 200 RU. The antigen was diluted with PBS-T running buffer by concentration and injected at a flow rate of 30 µl/min for 4 minutes, and the dissociation time was set to 10 minutes. To remove the fusion protein bound every cycle, a regeneration solution (10 mM glycine-HCl, pH 1.5) was injected at a flow rate of 30 µl/min for 1 minute. The experimental results were calculated using the 1:1 binding model of BIA evaluation software version 1.0. The binding ability is shown in Table 4 below, and SPR sensorgrams are shown in FIGs. 3a and 3b.

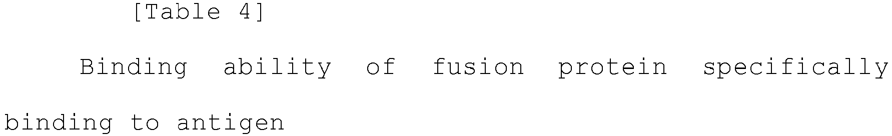

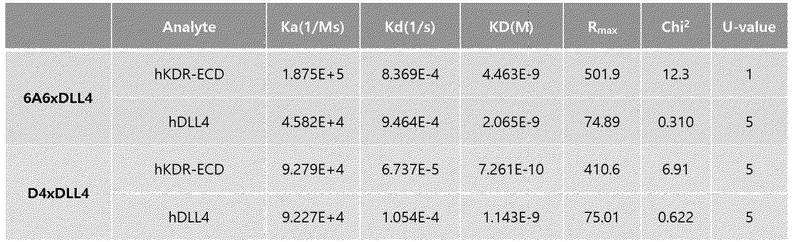

### Example 8. Confirmation of simultaneous binding ability of fusion protein to antigens

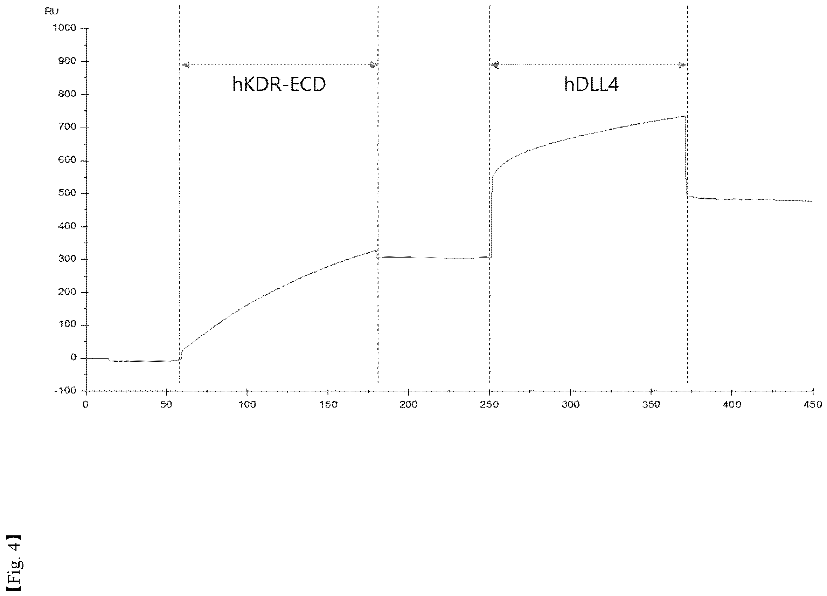

The ability of the selected fusion protein to simultaneously bind to two antigens was confirmed using SPR (Biacore T200, Cytiva). Using a Protein A chip, the fusion protein was immobilized on the surface according to the manufacturer's instructions. The fusion protein on the sensor chip was diluted with PBS-T running buffer to a concentration of 5 µg/ml to reach about 200 RU. Specifically, 100 nM hKDR-ECD diluted with PBS-T running buffer was injected at a flow rate of 30 µl/min to bind for 120 seconds, and then allowed to flow at a flow rate of 30 µl/min for 60 seconds using PBS-T running buffer. 200 nM hDLL4 diluted in PBS-T running buffer without regeneration was injected at a flow rate of 30 µl/min to bind for 120 seconds, and then allowed to flow at a flow rate of 30 µl/min for 60 seconds using PBS-T running buffer. The results of simultaneous binding to the two antigens are shown in FIG. 4.

### Example 9. Confirmation of HUVEC proliferation ability of fusion protein

In order to compare changes in the ability of the produced fusion protein to proliferate vascular endothelial cells (human umbilical vein endothelial cells (HUVECs)), vascular endothelial cells (human umbilical vein endothelial cells (HUVECs)) were treated with the fusion protein and comparative control antibodies (6A6, 6A6xDLL4, D4, Minod-Fc). 6A6xDLL4 is as shown in Table 5 below. Minod-Fc is a protein with Notch1 of SEQ ID NO: 18 and Fc fused.

**[Table 5]**

| **Composition** | | **Sequence** |
|---|---|---|
| 6A6x DLL4 | **Heavy chain region** | QMQLVQSGAEVKKPGASVKLSCKASGYTFSSYWMHWVRQAPGQ RLEWMGEINPGNGHTNYNEKFKSRVTITVDKSASTAYMELSSLRS EDTAVYYCAKIWGPSLTSPFDYWGQGTL ( SEQ ID NO: 35) |
| | **Light chain region** | DVDECSLGANPCEHAGKCINTLGSFECQCLQGYTGPRCEIDVNEC VSNPCQNDATCLDQIGEFQCICMPGYEGVHCESGGGGSGGGGSGS NFMLTQPPSVSVSPGKTARITCRGDNLGDVNVHWYQQRPGQAPV LVMYYDADRPSGIPERFSGSNSGNTATLTISGVEAGDEADYYCQV WDRTSEYVFGTGTKVTVLG (SEQ ID NO: 36) |

Specifically, HUVECs (LONZA, Cat No. C2519A) were cultured in a plate coated with 2% gelatin using VascuLife medium complete kit (Lifeline Cell Technology, Cat No. #LCT-LL-0005) . The culture was incubated at 37°C and 5% CO₂. HUVECs used in assay were cells within passage 10, and 1×10⁴ cells/well were inoculated into a 96-well plate with M199 medium (Gibco, Cat No. #11043-023) containing 0.5% heat inactivated FBS. Each antibody was diluted to 250 nM, 50 nM, 10 nM, and 2 nM, added to the corresponding well of the assay plate, and incubated for 30 minutes. Thereafter, VEGF165 (R&D Systems, Cat No. 293-VE) was treated at a concentration of 100 ng/ml per well. The test groups including a negative control group (VEGF-/Ab-) not treated with VEGF and a drug and a positive control group (VEGF+/Ab-) treated only with VEGF were cultured in a CO₂ incubator at 37°C for 3 days. Viability was measured by detecting ATP in living cells using a CellTiter Glo-Luminescent Cell viability assay kit (Promega, Cat No. G7571).

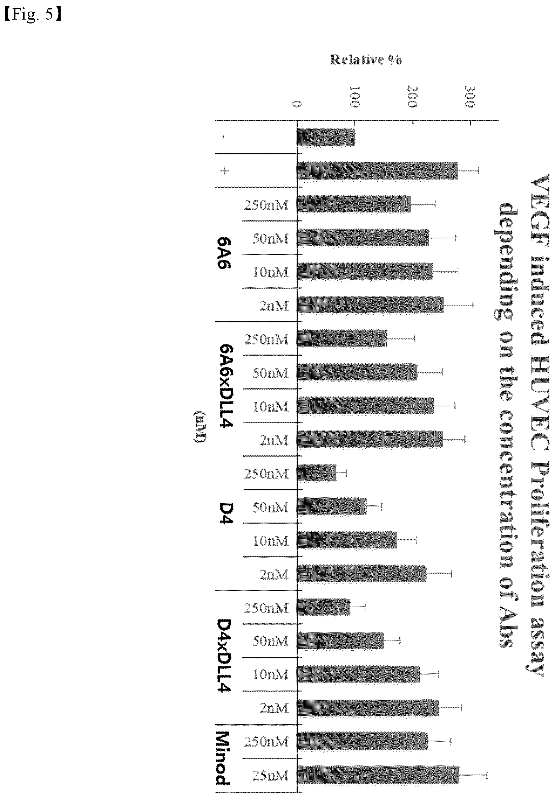

The test results showed that, compared to the 6A6 comparative group and the 6A6xDLL4 comparative group with a 6A6 backbone, the optimized antibody D4 comparative group and the D4xDLL4 test group with a D4 backbone inhibited the VEGF165-mediated HUVEC proliferation ability to a great extent, all of which were concentration-dependent. The above results are shown in FIG. 5.

### Example 10. Changes in inhibition of VEGFR-2 phosphorylation by fusion protein

To confirm the ability of the fusion protein to dephosphorylate KDR (VEGFR-2), the fusion protein and control antibodies (6A6, 6A6xDLL4, D4, Minod-Fc) were compared by Western blot analysis.

Specifically, HUVEC (LONZA, Cat No. C2519A) culture was inoculated at a concentration of 8×10⁵ cells/well into a 6-well plate using VascuLife medium complete kit (Lifeline Cell Technology, Cat No. #LCT-LL-0005), followed by culture for one day at 37°C and 5% CO₂. After treating HUVECs with each antibody at 125 nM and 25 nM for 30 minutes, each well was treated with VEGF165 (R&D, Cat No. 293-VE) at a concentration of 50 ng/ml for 10 minutes. The test groups included a negative control group (VEGF-/Ab-) not treated with VEGF and a drug and a positive control group (VEGF+/Ab-) treated only with VEGF. HUVECs treated with the drug were washed with phosphate-buffered saline, after which lysis buffer (Thermo Scientific, RIPA buffer & phosphatase inhibitors) was added, followed by cell lysis on ice for 10 minutes. The lysed cells were collected using a cell scraper and then centrifuged at 13,000 rpm at 4°C for 10 minutes to obtain only the supernatant. The total protein of the supernatant thus obtained was quantified using a BCA protein quantification method, and then 20 µg each was loaded onto a 4-15% grade SDS-PAGE gel and subjected to electrophoresis. After completion of electrophoresis, the protein was transferred to a nitrocellulose membrane (Bio-Rad, Trans-Blot Turbo Transfer System) for Western blot analysis. Blocking was performed at room temperature for 1 hour by adding blocking buffer [5% skim milk/TBS-T (0.1% Tween20, Tris-based saline)] to the nitrocellulose membrane blot. After washing with TBS-T buffer, the primary antibody (p-VERFR2 (Y1175), cell signaling) was diluted 1:1000, allowed to react at room temperature for 1 hour, and washed. The secondary antibody (anti-rabbit-HRP) was diluted 1:5000 and allowed to react at room temperature for 1 hour. After washing with TBS-T buffer, reaction was performed with ECL (enhanced chemiluminescence) solution (Thermo Fisher Scientific, Cat No. 34096), and images were obtained using a luminescent image analyzer (GE Healthcare, Amersham Imager 680).

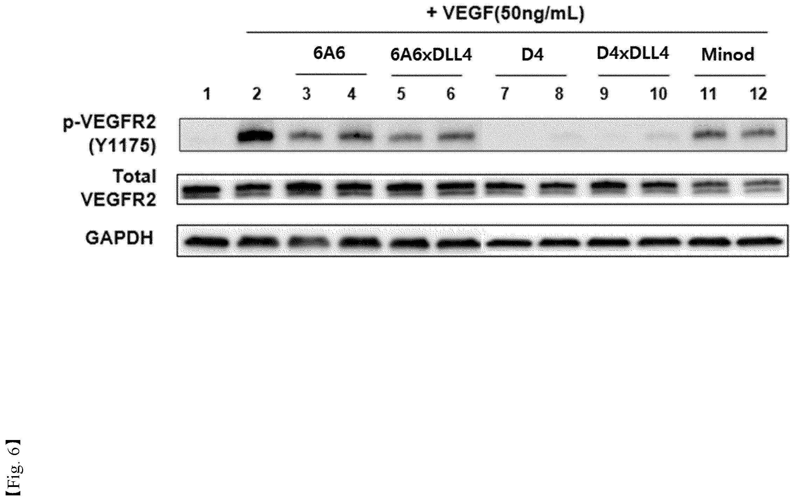

The test results showed that VEGF165-mediated VEGFR2 phosphorylation was partially inhibited in a concentration-dependent manner in the 6A6 comparative group and the 6A6xDLL4 comparative group, whereas in the optimized antibody D4 comparative group and the test group treated with D4xDLL4 in equal amounts, VEGFR2 was almost completely dephosphorylated. These results are shown in FIG. 6.

Example 11. Confirmation of KDR (VEGFR-2) binding ability using FACS

To determine the binding activity of the fusion protein to HUVECs, FACS analysis was performed using the fusion protein and control antibodies (6A6, 6A6xDLL4, D4, Minod-Fc).

Specifically, HUVECs (LONZA, Cat No. C2519A) were cultured at 37°C and 5% CO₂ using a plate coated with 2% gelatin and VascuLife medium complete kit (Lifeline Cell Technology, Cat No. #LCT-LL-0005). The cells were prepared at a concentration of 2×10⁷ cells/ml in FACS buffer (0.5% FBS, 0.05% sodium azide in PBS) and dispensed at 50 pl into each of FACS tubes. 100 pl of each antibody at a concentration of 5 nM was mixed in the FACS tube, followed by reaction at 4°C for 30 minutes. After completion of reaction, 2 ml of FACS buffer was added to each tube, followed by centrifugation at 1500 rpm for 3 minutes. The supernatant was removed, the cells were resuspended, and 100 pl of anti-human IgG-PE antibody (1:200, Bethyl, #A80-248PE) was added, followed by reaction at 4°C for 20 minutes. 2 ml of FACS buffer was added to each FACS tube, followed by centrifugation at 1500 rpm for 3 minutes. The separated supernatant was removed, the cells were resuspended in 200 pl of FACS buffer, and the binding activity was analyzed using FACS (Beckton Dickinson, Lyric).

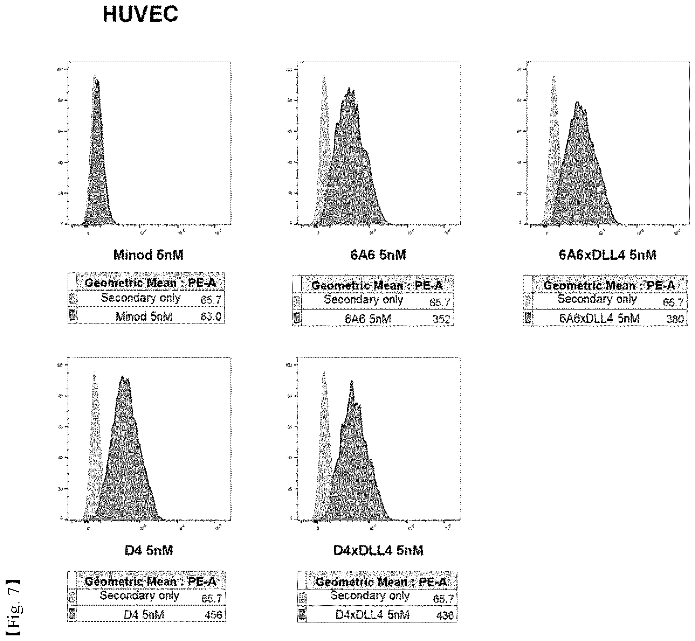

The test results showed that, excluding Minod-Fc, all 6A6, 6A6xDLL4, D4, and D4xDLL4 targeting VEGFR2 bound to HUVECs. Also, D4 and D4xDLL4 exhibited improved binding activity compared to 6A6 and 6A6xDLL4. These results are shown in FIG. 7.

### Example 12. Confirmation of binding ability of hDLL4 expressed on cell surface and antibody

To produce cells expressing DLL4 on the cell surface, HEK293 cells were transformed with pcDNA-hDLL4.

Specifically, the pcDNA-hDLL4 vector synthesized by GeneArt was transfected into HEK293 cells using Lipofectamin^{™} 2000 (Invitrogen #1168-019, USA), and neomycin (Gibco #10131, 50 mg/ml) was added for selection. Then, all grown cells were collected and cultured in a pool state. Then, 23 types of single colonies were obtained by culture using a limiting dilution method, and among the single colonies, one type of cell line that highly overexpressed hDLL4 was selected and tested. The selected cells were prepared to a concentration of 2×10⁷ cells/ml in FACS buffer (0.5% FBS, 0.05% sodium azide in PBS) and dispensed at 50 pl into each of FACS tubes. 5 nM fusion protein and control antibodies (6A6, 6A6xDLL4, D4, D4xDLL4, Minod-Fc) were added in 100 pl to each of the FACS tubes containing the cells, followed by reaction at 4°C for 30 minutes. After completion of reaction, 2 ml of FACS buffer was added to each tube, followed by centrifugation at 1500 rpm for 3 minutes. The supernatant was removed, the cells were well resuspended, and then 100 pl of anti-human IgG-PE antibody (1:200, BioLegend, #A80-248PE) was added, followed by reaction at 4°C for 20 minutes. After completion of reaction, 2 ml of FACS buffer was added to each FACS tube, followed by centrifugation at 1500 rpm for 3 minutes. The separated supernatant was removed, the cells were resuspended in 200 pl of FACS buffer, and the binding activity was analyzed using FACS (Beckton Dickinson, Lyric).

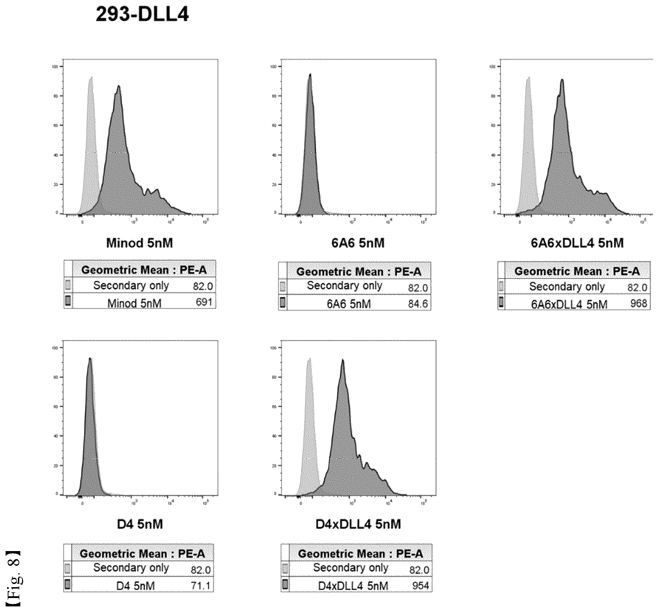

The test results showed that, only Minod-Fc, 6A6xDLL4, and D4xDLL4, including the Minod domain targeting DLL4, bound to DLL4-overexpressed HEK293 cells, and high binding appeared in the fusion proteins 6A6xDLL4 and D4xDLL4 rather than Minod-Fc alone. 6A6 and D4 did not bind to DLL4-overexpressed HEK293. These results are shown in FIG. 8.

### [Industrial Applicability]

According to the present invention, there is provided a fusion protein that simultaneously targets VEGFR2/KDR and DLL4, which includes an antibody with improved affinity compared to existing antibodies targeting VEGFR2/KDR, making it possible to provide co-therapy and monotherapy that are more effective than existing therapeutic agents in treating tumors. The fusion protein can exhibit increased binding ability to VEGFR2/KDR, and can be useful in the prevention or treatment of target cancer/tumors or diseases related to angiogenesis inhibitors. By simultaneously inhibiting the activity of DLL4 by Notch1, the fusion protein has higher angiogenesis inhibition ability than single treatment.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A fusion protein comprising an antibody binding to VEGFR2/KDR or an antigen-binding fragment thereof; and a DLL4 (delta-like ligand 4) binding protein domain,
wherein the antibody binding to VEGFR2/KDR or the antigen-binding fragment thereof comprises a heavy chain variable region comprising a heavy chain CDR1 of SEQ ID NO: 1, a heavy chain CDR2 of SEQ ID NO: 2, and a heavy chain CDR3 of SEQ ID NO: 3, and a light chain variable region comprising a light chain CDR1 of SEQ ID NO: 4, a light chain CDR2 of SEQ ID NO: 5, and a light chain CDR3 of SEQ ID NO: 6.

2. The fusion protein according to claim 1, wherein the antibody binding to VEGFR2/KDR comprises a heavy chain variable region of SEQ ID NO: 8 and a light chain variable region of SEQ ID NO: 10.

3. The fusion protein according to claim 1, wherein the DLL4 binding protein is Notch1 comprising a sequence of SEQ ID NO: 18.

4. The fusion protein according to claim 1, wherein the DLL4 binding protein domain binds to an N terminus of a light chain of the antibody binding to VEGFR2/KDR.

5. The fusion protein according to claim 1, wherein the antibody binding to VEGFR2/KDR or the antigen-binding fragment thereof and the DLL4 binding protein domain are connected through a linker.

6. The fusion protein according to claim 1, wherein a linker comprises (GnS)m (in which n and m are each from 1 to 10).

7. A nucleic acid encoding the fusion protein according to any one of claims 1 to 6.

8. An expression vector comprising the nucleic acid according to claim 7.

9. A transformed cell comprising the expression vector according to claim 8.

10. A method of producing a fusion protein, comprising:
(a) producing a fusion protein by culturing the cell according to claim 9; and
(b) recovering the produced fusion protein.

11. A composition for preventing or treating an angiogenic disease comprising the fusion protein according to any one of claims 1 to 6 as an active ingredient.

12. A composition for diagnosing an angiogenic disease comprising the fusion protein according to any one of claims 1 to 6 as an active ingredient.

13. A composition for preventing or treating a tumor or cancer comprising the fusion protein according to any one of claims 1 to 6 as an active ingredient.

14. A composition for co-administration with an additional therapeutic agent comprising the fusion protein according to any one of claims 1 to 6.
